# EUROPÄISCHE PATENTSCHRIFT

(11) **EP 0 737 069 B1**
(45) Veröffentlichungstag und Bekanntmachung des Hinweises auf die Patenterteilung: **16.04.2003**
(21) Anmeldenummer: 95902785.5
(22) Anmeldetag: 06.12.1994
(51) Int. Cl.: A61K 31/485, A61K 31/19, A61K 31/05, A61K 31/135, A61K 9/06

(54) **DEUTERIERTE WIRKSTOFFE IN TRANSDERMALER APPLIKATION**
DEUTERISED ACTIVE AGENTS IN TRANSDERMAL APPLICATION
SUBSTANCES ACTIVES DEUTEREES EN APPLICATION TRANSDERMIQUE

(30) Priorität: 22.12.1993 DE 4343838
(43) Veröffentlichungstag der Anmeldung: 16.10.1996
(73) Patentinhaber: LTS Lohmann Therapie-Systeme AG, 56626 Andernach (DE)
(72) Erfinder: HOFFMANN, Hans-Rainer, D-56567 Neuwied (DE); HILLE, Thomas, D-56567 Neuwied (DE); KOCH, Andreas, D-56581 Melsbach (DE); MATUSCH, Rudolf, D-35041 Marburg (DE)
(74) Vertreter: Flaccus, Rolf-Dieter, Dr.
(86) Internationale Anmeldenummer: EP9404047
(87) Internationale Veröffentlichungsnummer: WO95017188

(56) Entgegenhaltungen:
- EP-A- 0 307 786
- US-A- 4 473 585
- US-A- 4 529 600
- US-A- 5 075 337
- ALDRICH'S CATALOGUE HANDBOOK OF FINE CHEMICALS, 1992
- SIGMA'S BIOCHEMICALS AND ORGANIC COMPOUNDS FOR RESEARCH AND DIAGNOSTICS, 1992
- STN INTERNATIONAL, KARLSRUHE

## Beschreibung

Die Erfindung betrifft die transdermale Applikation von deuterierten Arzneistoffen. Den zweifellos großen Vorteilen, die die transdermale Applikation von Pharmaka aufweist, steht oft als Nachteil die Limitierung der Menge des Arzneistoffs gegenüber, die über die Haut aufgenommen werden kann. Daher wurde gleichzeitig mit Beginn der Therapie durch dermale Applikation nach Wegen gesucht, die Penetrationsfähigkeit von Pharmaka durch die Haut zu erhöhen.
Ein Lösungsansatz wurde zunächst in der Entwicklung von Penetrationsförderern gesehen, die Arzneimitteln zur dermalen Applikation zugefügt werden. Diese Substanzen verändern zumindest für einen kurzen Zeitraum tieferliegende Hautstrukturen und können in ungünstigen Fällen zu unerwünschtem Nebeneffekten führen. Andere Möglichkeiten zur Erhöhung der Resorption von Wirkstoffen bestehen im Entfernen des Stratum corneum durch Laserstrahlbehandlung oder durch wiederholtes Aufkleben und Abreißen von Klebestreifen, dem sog. "stripping". Bei diesem Verfahren ist nachteilig, daß nicht nur das erwünschte Penetrieren des Wirkstoffs, sondern auch aller anderen Stoffe sowie von Mikroorganismen, wie Bakterien und Pilzsporen, in den menschlichen Körper erleichtert wird.
Ein weiterer Weg, die dermale Resorption zu verbessern, besteht in der Anwendung von Strom. Dieses unter dem Begriff "Iontophorese" bekannte Verfahren kann, wie dem medizinischen Fachmann bekannt ist, nicht schmerzfrei angewandt werden. Ebenfalls nicht schmerzfrei angewandt werden kann das sog. "Stachelpflaster". Diese Arzneiform wird mit Kanülen, die die Hornhaut durchdringen, am Körper fixiert. Die Wirkstoffabgabe erfolgt durch die Kanülen, die gleichzeitig als Fixierhilfe dienen.

Eine auf den ersten Blick interessante Alternative stellt die dermale Verabreichung sogenannter "pro-drugs" dar. Hierbei werden Strukturelemente von Arzneistoffen, die als besonders ungünstig für die dermale Resorption gelten, z.B. phenolische Hydroxylgruppen, derivatisiert, z.B. verestert. Das Kennzeichen der chemischen Abwandlung der pro-drugs ist, daß die Derivatisierung äußerst labil ist, so daß in vitro rasch und vollständig das dem pro-drug zugrundeliegende Pharmakon entsteht. Dem Fachmann ist jedoch bekannt, daß dieser an sich elegante Lösungsgedanke in der Praxis selten verwirklicht werden kann, denn in vivo findet diese angestrebte rasche und vollständige Metabolisierung nicht statt. Damit ergeben sich toxikologische Fragestellungen zu den pro-drugs, die noch weitreichende pharmakologische Untersuchungen erfordern.

In der Vergangenheit wurden versuchsweise radioaktiv markierte Arzneistoffe transdermal appliziert, um die oft nach transdermaler Gabe äußerst niedrigen Blutspiegel leichter erfassen zu können; ein Austausch des radioaktiven Bestandteils unter in vivo-Bedingungen sollte dabei nicht stattfinden.

Aufgabe der Erfindung ist daher die Bereitstellung eines Arzneimittels zur transdermalen Gabe von Wirkstoffen, das eine erhöhte Resorptionsrate für Wirkstoffe ermöglicht, wobei die Nachteile der im Stand der Technik bekannten Verfahren vermieden werden. Diese Aufgabe wird erfindungsgemäß in überraschender Weise gelöst durch ein Arzneimittel zur Abgabe von Wirkstoffen an die Haut mit einem gegenüber der natürlichen Isotopenverteilung erhöhten Deuteriumanteil.

Diese Lösung ist umso überraschender, als deuterierte Verbindungen eigentlich aufgrund der Zunahme ihrer Molmasse im Vergleich zu den entsprechenden nicht deuterierten Substan-zen ein schlechteres bzw. im günstigsten Fall allenfalls gleiches Diffusioneverhalten zeigen sollten.

Unter deuterierten Verbindungen im Sinne der Erfindung werden Verbindungen verstanden, in denen mindestens ein oder mehrere Wasserstoffatome durch Deuterium ersetzt sind.

Gegenstand der Erfindung sind nicht isotopenmarkierte Verbindungen, die für Untersuchungen zur Biosynthese und zum Bioabbau von Naturstoffen, für Arbeiten über den Metabolismus von Pharmaka und anderen Chemikalien in pflanzlichen und tierischen Organismen benötigt werden, da diese stabil sein müssen. Bei der vorliegenden Erfindung kann jedoch durchaus eine Rücktauschreaktion stattfinden in der Weise, daß die eingeführten Deuteriumatome wieder im Körper durch eine Austauschreaktion durch Wasserstoffatome ersetzt werden. Dem Fachmann ist bekannt, daß Wasserstoff immer ein Isotopengemisch aus ¹H, ²D und ³T darstellt. Daher liegen alle chemischen Verbindungen, die Wasserstoff enthalten, immer im Gemisch aus deuterierten und nicht deuterierten Verbindungen vor, wobei der Anteil an ²D bei 0,015 Mol-% liegt. Eine bevorzugte Ausgestaltung der Erfindung sind Gemische, bei denen der Anteil an deuterierten Verbindungen erheblich, d.h. mindestens auf 10 Mol-%, erhöht ist. Weiterhin bevorzugt sind solche Arzneimittel, bei denen der Anteil an Deuterium, bezogen auf austauschbare Wasserstoffisotope, mindestens 10 Mol-% beträgt.

Dem Fachmann ist der Begriff Arzneimittel bekannt. Zur Applikation auf der Haut oder auf Schleimhäuten (z.B. Nase, Auge) sind Salben, die Gele von plastischer Verformbarkeit darstellen, geeignet, ebenso Pasten, die als Salben mit hohem Feststoffanteil bezeichnet werden können. Aus naheliegenden Gründen dürfen diese Arzneiformen keine protischen Lösemittel wie z.B. Wasser oder Ethanol enthalten.

Unter einem transdermalen therapeutischen System (TTS) soll nach Zaffaroni "eine arzneistoffenthaltende Vorrichtung bzw. eine Darreichungsform, die einen Arzneistoff oder mehrere in vorausbestimmter Rate kontinuierlich über einen festgesetzten Zeitraum an einen festgelegten Anwendungsort abgibt" (zitiert nach Heilmann, Therapeutische Systeme - Konzept und Realisation programmierter Arzneiverabreichung, 4. Auflage, Ferdinand Enke Verlag Stuttgart 1984, S. 26, wobei im vorliegenden Fall der Anwendungsort die Haut ist.

Der Aufbau von trandermalen Systemen ist dem Fachmann bekannt. Schutzrechte, in denen der grundsätzliche Aufbau beschrieben wird, sind beispielsweise DE 33 15 272, DE 38 43 239, US 3,598,122.

Wird ein transdermales therapeutisches System auf die Haut eines Patienten appliziert, so soll der Arzneistoff abgegeben werden, um beim Patienten topisch oder systemisch wirksam zu werden. Arzneiformen dieser Art werden bereits therapeutisch genutzt. Sie sind meist schichtenförmig aufgebaut und bestehen im einfachsten Falle aus einer Rückschicht, einem selbstklebenden Wirkstoffreservoir und einer wiederablösbaren Schutzschicht, die vor der Applikation zu entfernen ist.

Als Wirkstoffe werden Substanzen verwendet, die ohne oder mit Resorptionsfilter auf der Haut appliziert, eine lokale oder systemische Wirkung hervorrufen.

Stoffe mit lokaler Wirkung sind z.B. Antitranspirantia, Fungizide, Bactericide und Bacteriostatica.

Stoffe mit systemischer Wirkung sind beispielsweise Antibiotika, Hormone, Antipyretica, Antidiabetica, Koronardilatatoren, herzwirksame Glycoside, Spasmolytika, Antihypertonica, Psychopharmaka, Migränemittel, Corticoide, Analgetica, Kontrazeptiva, Antirheumatica, Anticholinergica, Sympatolytica, Sympatomimetica, Vasodilatatoren, Anticoagulantien und Antiarrhythmika.

Damit eine Rücktauschreaktion im menschlichen Körper stattfinden kann, müssen solche Wasserstoffatome durch Deuterium ersetzt sein, die durch Behandlung mit Deuteriumoxid (Umkristallisieren, Ausschütteln etc.) austauschbar sind, z.B. Wasserstoffatome in O-H oder N-H Bindungen.

Die Erfindung wird durch folgende Beispiele erläutert:

### 1. Herstellung der deuterierten Verbindungen

### 1.1 Herstellung der deuterierten Morphinbase

100 mg Morphinbase-Monohydrat werden in der Siedehitze in 90 ml reinstem Deuteriumoxid gelöst. Man läßt auf Raumtemperatur erkalten, wobei sich allmählich, weiße, nadelförmige Kristalle bilden, die nach Abtrennung bis zur Gewichtskonstanz im Exsiccator getrocknet werden.
Ausbeute: 64 mg = 64 % d. Theorie Schmelzbereich: ab 235°C (Morphinbase: ab 235°C)

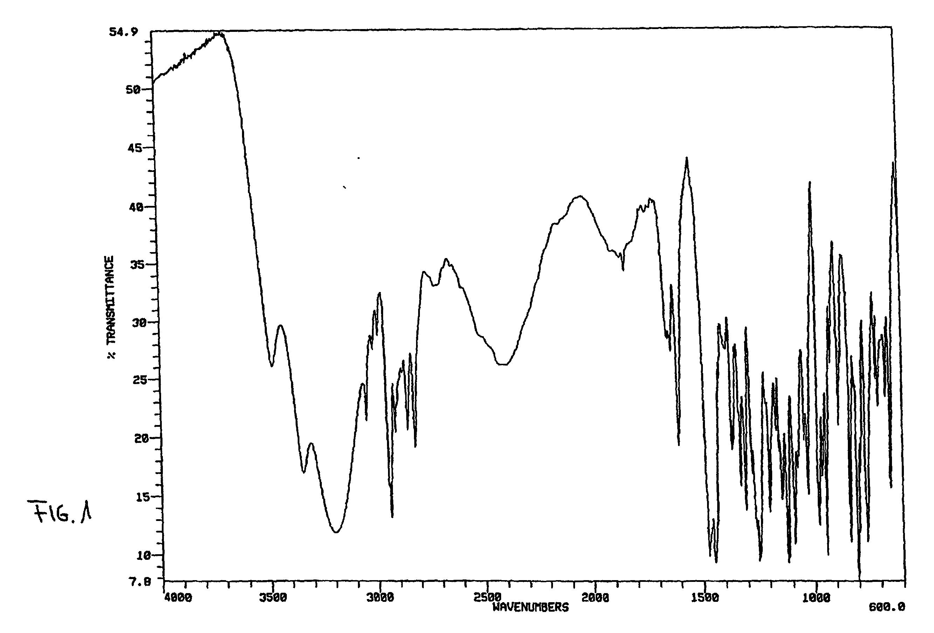

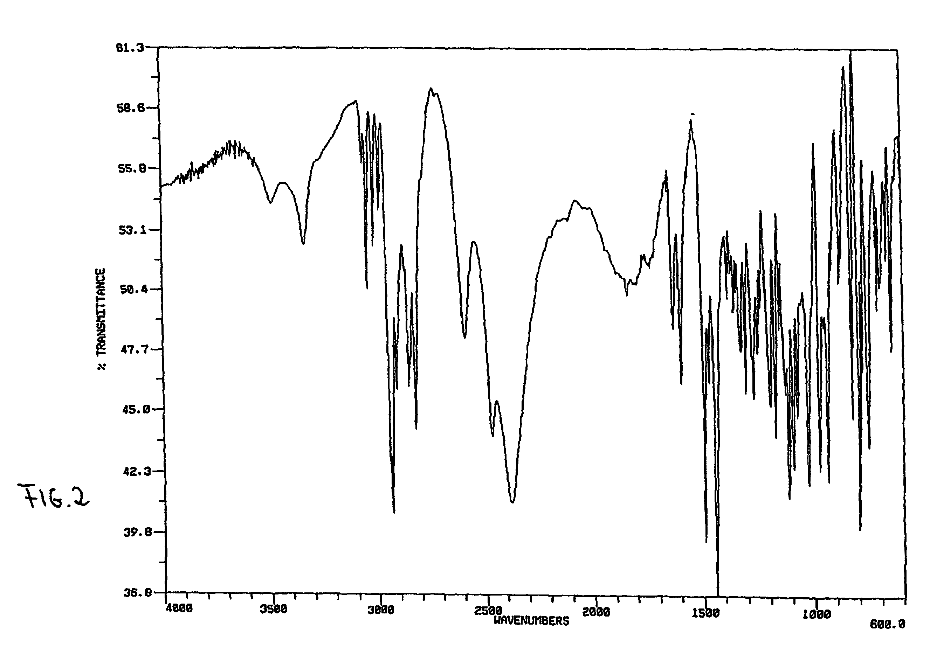

Die IR-Spektren von Edukt und Produkte sind in Fig. 1 und 2 abgebildet.

Aufgrund der Banden der O-H bzw. O-D Valenzschwingungen erkennt man, daß der Deuterierungsgrad, bezogen auf austauschbare Wasserstoffatome ca. 95% beträgt.

### 1.2. Herstellung von deuterierter Ölsäure

10 g Ölsäure entsprechend NF XVII gemäß USP XXII werden mit 40 g Deuteriumoxid vermischt und bei Raumtemperatur mehrere Stunden lang innig verrührt. Die Lösung wird dekantiert, und die Ölsäurephase wird im Vakuum getrocknet.

### 1.3 Herstellung von deuteriertem Salbutamol

0,5 g Salbutamol wird in 20 g Deuteriumoxid unter Erwärmen gelöst. Da der Stoff nicht auskristallisiert, dampft man unter Vakuum bei Raumtemperatur ein.
Ausbeute: 0,5 g Fp: 149,8°C (Fp Salbutamol 153,4 °C)

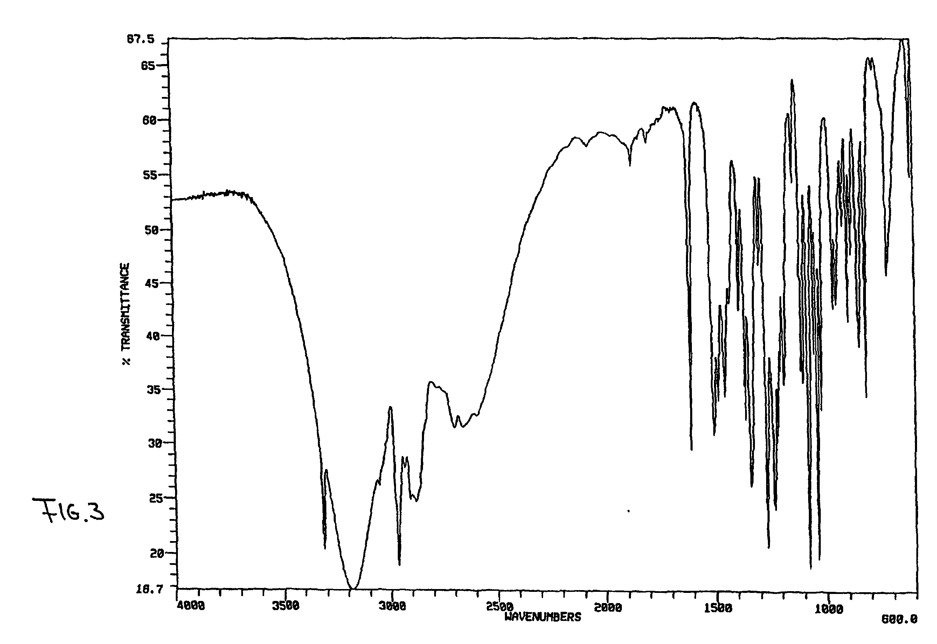

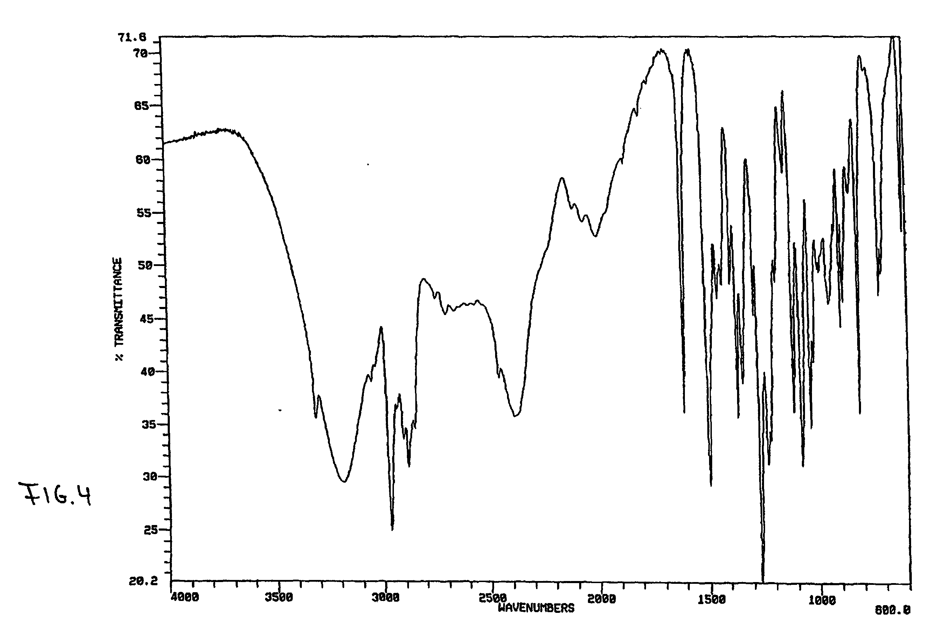

Die IR-Spektren von Edukt und Produkt sind in Fig. 3 und 4 abgebildet.

An Hand der IR-Spektren erkennt man, daß der Deuterierungsgrad, bezogen auf austauschbare Wasserstoffatome, nahezu 100% beträgt.

### 1.4 Herstellung von deuterierter Benzoesäure

2,0 g Benzoesäure werden aus 45 ml siedendem Deuteriumoxid umkristallisiert.
Ausbeute: 1,86 g Fp: 120,3°C (Fp Benzoesäure 122,8 °C)

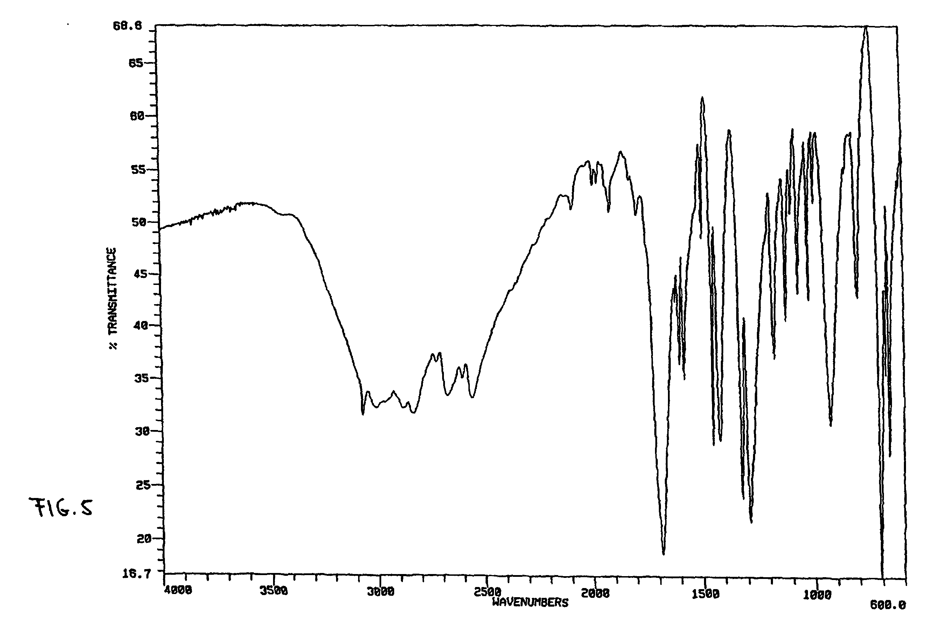

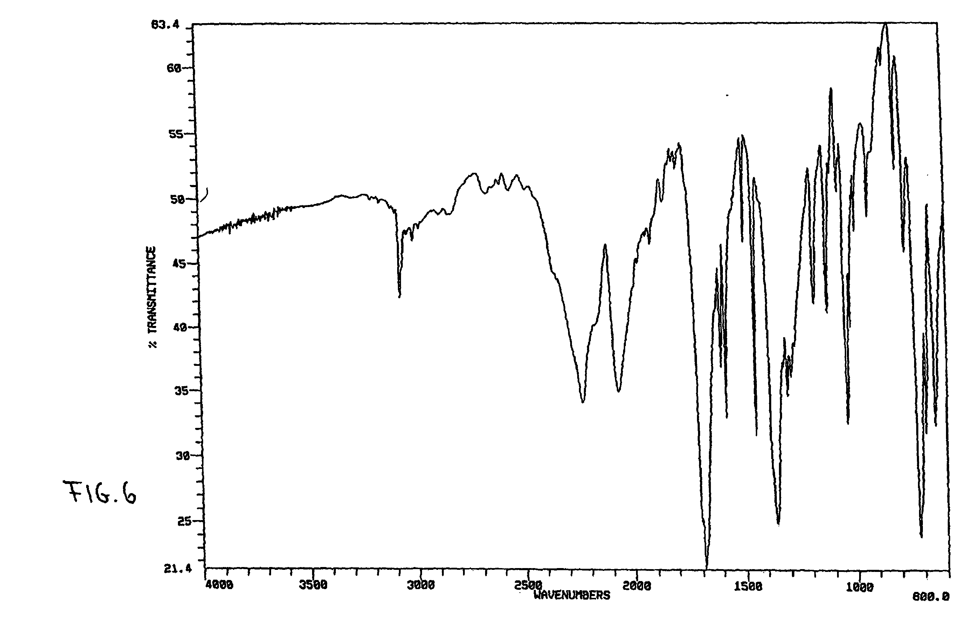

Die IR-Spektren von Edukt und Produkt sind in Fig. 5 und 6 abgebildet.

Man erkennt, daß der Deuterierungsgrad nahezu 100% beträgt.

### 1.5 Herstellung von deuteriertem Phenol

5,0 g Phenol werden mit 5,0 g Deuteriumoxid vermischt und erhitzt. Anschließend wird Deuteriumoxid im Wasser-Gemisch im Vakuum abgesogen.

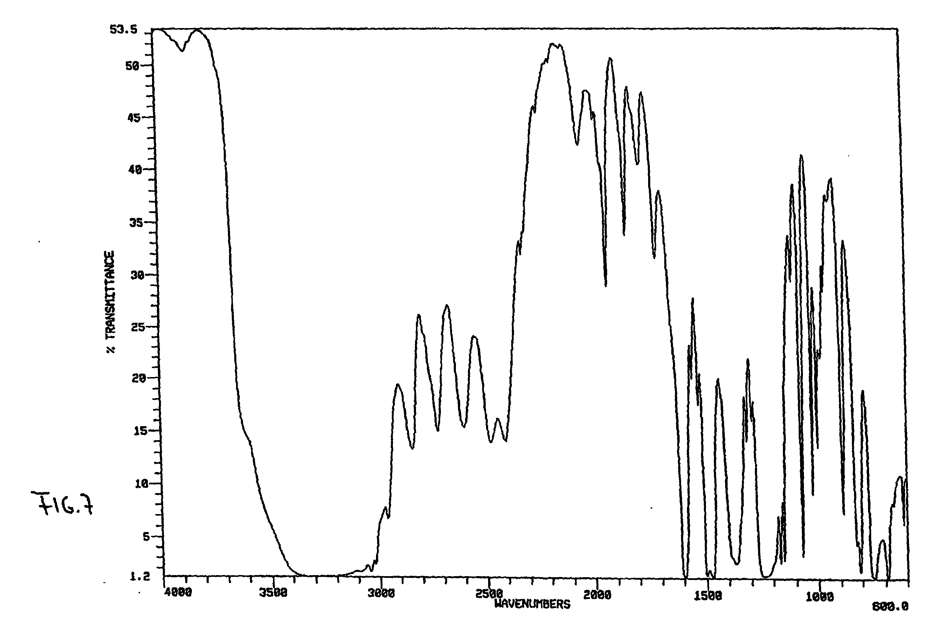

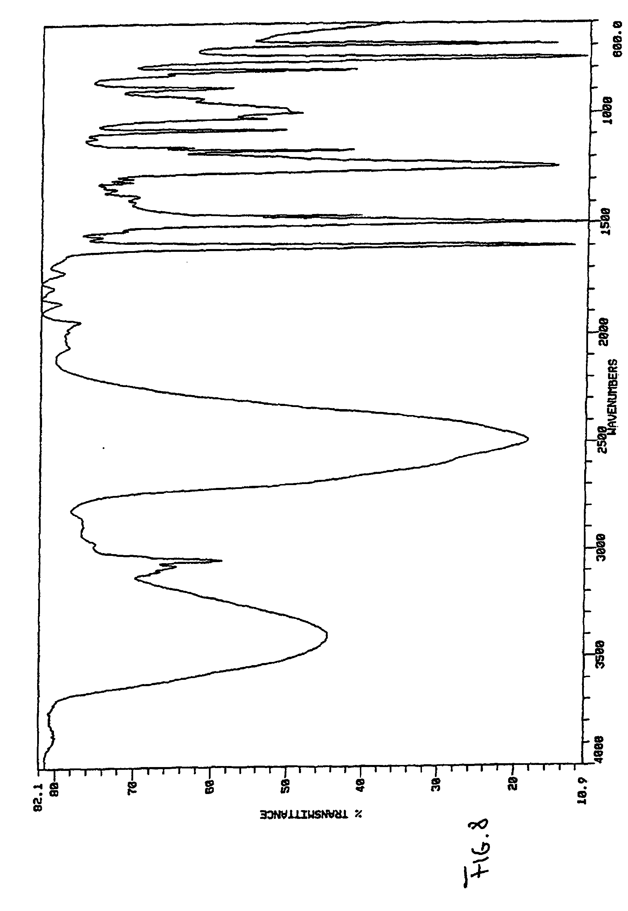

Die IR-Spektren von Edukt und Produkt sind in Fig. 7 und 8 abgebildet.

Man erkennt, daß der Deuterierungsgrad nahezu 100% beträgt.

Man erkennt aus den nachfolgend aufgeführten Spektren deutlich, daß die Banden, die durch die Valenzschwingungen der beiden Hydroxylgruppen hervorgerufen werden, zu niedrigeren Wellenzahlen verschoben wurden, was durch das höhere Atomgewicht von ²D gegenüber ¹H auch zu erwarten war. Der Nachweis des Austauschs läßt sich daher durch IR-Spektroskopie führen.
- Fig. 1: IR-Spektrum von Morphinbase-Monohydrat
- Fig. 2: IR-Spektrum von deuterierter Morphinbase
- Fig. 3: IR-Spektrum von Salbutamol
- Fig. 4: IR-Spektrum von deuteriertem Salbutamol
- Fig. 5: IR-Spektrum von Bezoesäure
- Fig. 6: IR-Spektrum von deuterierter Benzoesäure
- Fig. 7: IR-Spektrum von Phenol
- Fig. 8: IR-Spektrum von deuteriertem Phenol

### 2. In vitro-Permeation

### 2.1.1 Auflösen von Morphinbase

400 mg deuterierte Morphinbase (vgl. Bsp. 1.1.) werden in 1600 mg deuterierter Ölsäure (vgl. Bsp. 1.2) und 1 g deuterierten Methanol (D₃C-OD) gelöst (vgl. 1.2).

Man entfernt nach Auflösen das deuterierte Methanol im Vakuum.

Analog stellt man eine Morphin/Ölsäure-Lösung aus undeuterierten Verbindungen her.

### 2.1.2. Auflösen von Salbutamol

200 mg deuteriertes Salbutamol (vgl. Bsp. 1.3) werden in 800 mg deuterierter ölsäure (vgl. Bsp. 1.2.) und 2 g 2-Butanon gelöst. Man entfernt nach Auflösen das 2-Butanon im Vakuum (vgl. 1.2).

Analog stellt man eine Salbutamol/Ölsäure-Lösung aus undeuterierten Verbindungen her.

### 2.1.3. Auflösen von Benzoesäure

0,5 g deuterierte Benzoesäure (vgl. Bep. 1.4) werden in 4,5 g Dimethylisosorbit gelöst. Analog stellt man eine Lösung aus nicht deuterierter Benzoesäure her.

### 2.1.4 Auflösen von Phenol

0,5 g deuteriertes Phenol (vgl. Bsp. 1.5) werden in 4,5 g Dimethylisosorbit gelöst. Analog stellt man eine Lösung aus nicht deuteriertem Phenol her.

### 2.2 Penetrationsexperimente

Die Experimente werden an excidierter Meerschweinchenhaut durchgeführt, die in eine Franz'sche Diffusionszelle eingespannt wurde. Als Akzeptormedium dient physiologische Kochsalzlösung, die Gehaltsbestimnungen erfolgen per HPLC. Die Ergebnisse sind in Tab. 1 wiedergegeben.

Ergebnisse der Penetrationsexperimente

### 2.2.1 Salbutamol

**Tab. 1**

| Penetrationsergebnisse von Salbutamol und deuteriertem Salbutamol an excidierter Meerschweinchenhaut | | | | |
|---|---|---|---|---|
| | 3 h | 5 h [µg/cm²] | 7 h [µg/cm²] | 24 h [µg/cm² |
| Salbutamol | | 14 | 19 | 467 |
| deuteriertes Salbutamol | | 23,0 | 49,0 | 1067 |

### 2.2.2 Morphin

**Tab. 2**

| Penetrationsergebnisse von Morphin und deuteriertem Morphin an excidierter Meerschweinchenhaut | | | | |
|---|---|---|---|---|
| | 6 h [µg/cm²] | 18 h [µg/cm²] | 22 h [µg/cm²] | 26 h [µg/cm²] |
| Morphin | 2,6 | 84 | 153 | 256 |
| deut. Morphin | 5,3 | 103 | 187 | 299 |

Es ist zu erkennen, daß alle Werte der deuterierten Form einen erhöhten Flux belegen.

### 2.2.3 Benzoesäure

**Tab. 3**

| Penetrationsergebnisse von Benzoesäure und deuterierter Benzoesäure an excidierter Meerschweinchenhaut | | | | |
|---|---|---|---|---|
| | 2 h [µg/cm²] | 4 h [µg/cm²] | 8 h [µg/cm²] | 24 h [µg/cm²] |
| Benzoesäure | 54 | 252 | 1022 | 1700 |
| deut. Benzoesäure | 73 | 320 | 1249 | 2483 |

### 2.2.4 Phenol

**Tab. 4**

| Penetrationsergebnisse von Phenol und deuteriertem Phenol an excidierter Haut des Yucatan-Micro-Pigs. | | | | |
|---|---|---|---|---|
| | 2 h [µg/cm²] | 4 h [µg/cm²] | 8 h [µg/cm²] | 24 h [µg/cm²] |
| Phenol | 2 | 7,9 | 46,4 | 487 |
| deut. Phenol | 2 | 13 | 74 | 701 |

Die Auftragung auf die Haut des Yucatan-Micro-Pigs war erforderlich, da Phenol nekrotisch und keratolytisch wirkt. Durch diesen nivellierenden Effekt wäre eventuell kein Unterschied zwischen deuteriertem und nicht deuteriertem Phenol zu erkennen gewesen.

Anhand des erzielten Flux erkennt man nicht nur, daß eine erhöhte Permeationsrate durch den Ersatz von Wasserstoff gegen Deuterium erzielt werden kann, sondern auch, daß dieser Effekt umso größer ist, je mehr wasserstoffatome ausgetauscht wurden (vgl. Benzoesäure/Salbutamol).

## Patentansprüche

1. Arzneimittel zur Abgabe von Wirkstoffen an die Haut, **dadurch gekennzeichnet, daß** der Wirkstoff einen gegenüber der natürlichen isotopenverteilung erhöhten Deuteriumanteil, bezogen auf die austauschbaren Wasserstoffisotope, besitzt.

2. Arzneimittel nach Anspruch 1, **dadurch gekennzeichnet, daß** es ein Gemisch aus deuterierten und nichtdeuterierten Wirkstoffen enthält.

3. Arzneimittel nach Anspruch 2, **dadurch gekennzeichnet, daß** der Anteil an deuterierten Verbindungen mindestens 10 Mol-% beträgt.

4. Arzneimittel nach Anspruch 2, **dadurch gekennzeichnet, daß** der Anteil an Deuterium, bezogen auf austauschbare Wasserstoffisotope, mindestens 10 Mol-% beträgt.

5. Arzneimittel nach einem oder mehreren der Ansprüche 1 bis 4, **dadurch gekennzeichnet, daß** es sich um eine Salbe oder Paste handelt.

6. Arzneimittel nach einem oder mehreren der Ansprüche 1 bis 4, **dadurch gekennzeichnet, daß** es sich um ein traxisdermales therapeutisches System handelt.

7. Verfahren zur Herstellung eines Arzneimittels zur Wirkstoffabgabe durch die Haut, **dadurch gekennzeichnet, daß** ein Wirkstoff mit einem einen gegenüber der natürlichen Isotopenverteilung erhöhten Deuteriumanteil, bezogen auf die austauschbaren Wasserstoffisotope, eingearbeitet wird.

8. Verfahren nach Anspruch 7, **dadurch gekennzeichnet, daß** mindestens ein Wirkstoff mit einem Deuteriumanteil von größer als 0,015 Mol-%, vorzugsweise von größer als 10 Mol-%, bezogen auf den Anteil der austauschbaren Wasserstoffisotope, eingearbeitet wird.

## Claims

1. A drug for the release of active substances to the skin, **characterized in that** the active substance has an increased deuterium portion, relative to the exchangeable hydrogen isotopes, as compared to the natural isotope distribution.

2. The drug according to claim 1 **characterized in that** it comprises a mixture of deuterated and non-deuterated active substances.

3. The drug according to claim 2 **characterized in that** the portion of deuterated compounds amounts to at least 10 mole-%.

4. The drug according to claim 2 **characterized in that** the portion of deuterium, relative to exchangeable hydrogen isotopes, amounts to at least 10 mole-%.

5. The drug according to one or several of claims 1 to 4 **characterized in that** it is an ointment or a paste.

6. The drug according to one or several of claims 1 to 4 **characterized in that** it is a transdermal therapeutic system.

7. A process for the production of a drug for the active substance release through the skin, **characterized in that** an active substance is incorporated that has an increased deuterium portion, relative to the exchangeable hydrogen isotopes, as compared to the natural isotope composition.

8. A process according to claim 7, **characterized in that** at least one active substance is incorporated that has a deuterium portion of more than 0.015 mole-%, preferably more than 10 mole-%, relative to the portion of exchangeable hydrogen isotopes.

## Revendications

1. Médicament pour la libération de substances actives sur la peau, **caractérisé en ce que** la substance active possède une fraction de deutérium, rapportée à l'isotope d'hydrogène échangeable, supérieure à celle de la répartition naturelle des isotopes.

2. Médicament selon la revendication 1, **caractérisé en ce qu'**il contient un mélange de substances actives deutérées et non deutérées.

3. Médicament selon la revendication 2, **caractérisé en ce que** la fraction de composés deutérés représente au moins 10 moles %.

4. Médicament selon la revendication 2, **caractérisé en ce que** la fraction de deutérium, rapportée à l'isotope d'hydrogène échangeable, représente au moins 10 moles %.

5. Médicament selon une ou plusieurs des revendications 1 à 4, **caractérisé en ce qu'**il s'agit d'un onguent ou d'une pâte.

6. Médicament selon une ou plusieurs des revendications 1 à 4, **caractérisé en ce qu'**il s'agit d'un système thérapeutique transdermique.

7. Procédé pour la préparation d'un médicament pour la libération d'une substance active à travers la peau, **caractérisé en ce qu'**on met en oeuvre une substance active qui possède une fraction de deutérium, rapportée à l'isotope d'hydrogène échangeable, supérieure à celle de la répartition naturelle des isotopes.

8. Procédé selon la revendication 7, **caractérisé en ce qu'**on incorpore au moins une substance active dont la fraction de deutérium est supérieure à 0,015 mole %, de préférence supérieure à 10 moles %, rapportés à la fraction de l'isotope d'hydrogène échangeable.
